# EUROPÄISCHE PATENTSCHRIFT

(11) **EP 0 825 183 B1**
(45) Veröffentlichungstag und Bekanntmachung des Hinweises auf die Patenterteilung: **16.02.2000**
(21) Anmeldenummer: 97114470.4
(22) Anmeldetag: 21.08.1997
(51) Int. Cl.: C07D 211/74, C07D 221/20

(54) **Verfahren zur Herstellung von 1-Acetyl-4-Piperidonen**
Process for the preparation of 1-acetyl-4-piperidinones
Procédé pour la préparation de 1-acétyl-4-pipéridinones

(30) Priorität: 23.08.1996 DE 19634147
(43) Veröffentlichungstag der Anmeldung: 25.02.1998
(73) Patentinhaber: BASF AKTIENGESELLSCHAFT, 67056 Ludwigshafen (DE)
(72) Erfinder: Julius, Manfred, Dr., 67117 Limburgerhof (DE)
(74) Vertreter: Isenbruck, Günter, Dr.

(56) Entgegenhaltungen:
- DE-A- 3 800 987

## Beschreibung

Die Erfindung betrifft ein Verfahren zur Herstellung von 1-Acetyl-4-piperidonen der nachstehenden Formel I 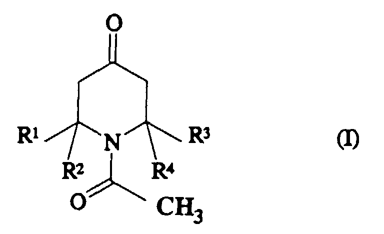 in der R¹, R², R³ und R⁴ eine C₁- bis C₆-Alkylgruppe
oder
R¹ und R² oder/und R³ und R⁴ jeweils gemeinsam eine alicylische Kohlenwasserstoffkette mit 2 bis 5 C-Atomen bedeuten; insbesondere betrifft die Erfindung ein Verfahren zur Herstellung von 1-Acetyl-2,2,6,6-tetramethyl-4-piperidon (N-Acetyl-triacetonamin; abgekürzt N-Acetyl-TAA).

N-Acetyl-TAA ist als Zwischenprodukt für die Synthese von Wärmestabilisatoren sowie von Stabilisatoren vom HALS-Typ (HALS = Hindered Amine Light Stabilizers) bekannt, die als Additive für die Stabilisierung von Kunststoffen wie etwa Polyolefinen eine wichtige Rolle spielen.

N-Acetyl-TAA kann durch Acetylierung von Triacetonamin mit Acetylchlorid oder mit Acetanhydrid hergestellt werden. Diese Art der Acetylierung von Triacetonamin ist jedoch schwierig und führt leicht zu Verharzungen.

Zur Lösung dieses Problems beschreibt die DE-C 38 00 987 ein Verfahren zur Herstellung von N-Acetyl-triacetonamin, bei dem Triacetonamin mit Acetanhydrid in einem Molverhältnis von 1:2,5 bis 1:10 bei Temperaturen von 80 bis 140°C umgesetzt wird und die anfallende Essigsäure sowie gegebenenfalls das Lösungsmittel kontinuierlich abdestilliert werden.

Bei diesem bekannten Verfahren ist jedoch der benötigte Acetanhydrid-Überschuß insofern nachteilig, als bei jedem Reaktionsansatz Acetanhydrid zurückgewonnen werden muß. Außerdem ist die hohe Reaktionstemperatur von in der Regel 110°C von Nachteil.

Neben diesen bekannten Acetylierungsmitteln kann auch Keten zur Acetylierung verwendet werden. Bei der Umsetzung von Keten als Acetylierungsmittel mit Carbonylverbindungen kommt es zur Bildung von Beta-Lactonen oder Enolacetaten. Aus der DE-B 1 121 605 ist bekannt, bei der Umsetzung von Carbonylverbindungen mit Keten die Bildung von Enolacetaten dadurch zu bevorzugen, daß die enolisierbare Carbonylverbindung, z.B. Aceton bei Temperaturen von 20 bis 110°C in Gegenwart eines phosphorsauren Katalysators reagieren gelassen wird. Bei dem phosphorsauren Katalysator handelt es sich um Difluorphosphorsäure oder ein Gemisch aus äquimolaren Mengen an Difluorphosphorsäure und Monofluorphosphorsäure oder das Reaktionsprodukt aus Phosphorpentoxid mit Fluorwasserstoff. Unter diesen Reaktionsbedingungen werden über 80 % der Carbonylverbindung zu den entsprechenden O-acetylierten Enolen umgesetzt.

Aufgabe der vorliegenden Erfindung ist es, ein einfaches und kostengünstiges Verfahren zur Herstellung von 1-Acetyl-4-piperidonen der genannten Formel I bereitzustellen, bei dem das 4-Piperidon am Stickstoffatom acetyliert wird und gleichzeitig die Herstellung von O-acetylierten Enolen vermieden wird.

Diese Aufgabe wird dadurch gelöst, daß ein Verfahren zur Herstellung von 1-Acetyl-4-piperidonen der nachstehenden Formel I 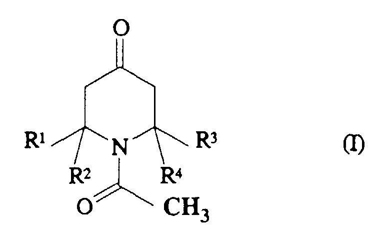 in der R¹, R², R³ und R⁴ eine C₁- bis C₆-Alkylgruppe
oder
R¹ und R² oder/und R³ und R⁴ jeweils gemeinsam eine alicyclische Kohlenwasserstoffkette mit 2 bis 5 C-Atomen bedeuten, bereitgestellt wird, das dadurch gekennzeichnet ist, daß ein das entsprechende nicht-acetylierte 4-Piperidon enthaltender Bestandteil mit Keten in Gegenwart einer Säure mit einem pK_{S}-Wert < 4,7 als Katalysator bei 20°C bis 120°C umgesetzt wird.

Kernpunkt des erfindungsgemäßen Verfahrens ist es, daß es bei der Umsetzung von Keten mit nicht-acetylierten 4-Piperidonen, insbesondere mit Triacetonamin in Gegenwart eines sauren Katalysators nicht zu der in der Literatur allgemein für Carbonylverbindungen beschriebenen Bildung von O-acetylierten Enolen kommt. Vielmehr ist unter diesen speziellen Reaktionsbedingungen die Acetylierung des Triacetonamins am Stickstoffatom bevorzugt. Ein weiterer Vorteil des erfindungsgemäßen Verfahrens ist seine ausgezeichnete Selektivität, die über 90% beträgt.

Als Katalysator wird bei dem erfindungsgemäßen Verfahren ein saurer Katalysator mit einem pK_{S}-Wert von weniger als 4,7, insbesondere Difluorphosphorsäure verwendet. Der Gehalt an diesem Katalysator, bezogen auf den Ausgangsstoff, beträgt bevorzugt 0,1 bis 10 mol-%, bevorzugt 1 bis 5 mol-%. Im Falle der Verwendung von Difluorphosphorsäure ist ein Gehalt von 1 bis 3 mol-% am stärksten bevorzugt.

Als Lösungsmittel für die Reaktion kommen polare oder unpolare, inerte, aprotische, organische Lösungsmittel, z.B. Kohlenwasserstoffe oder Ether in Betracht. Bevorzugt werden Ether eingesetzt, insbesondere Tetrahydrofuran. Falls notwendig, so kann die Reaktion jedoch auch ohne Lösungmittel durchgeführt werden.

Wie bereits erwähnt verläuft die Reaktion sehr selektiv, insbesondere wenn die starke Säure Difluorphosphorsäure verwendet wird. Es können jedoch auch andere starke Säuren wie etwa Hexafluorphosphorsäure, Schwefelsäure, Phosphorsäure, Chlorsulfonsäure, p-Toluolsulfonsäure, Spuren von Wasser enthaltendes Bortrifluorid, d.h. also Fluorwasserstoffsäure verwendet werden. Dies ist ganz besonders überraschend, da unter vergleichbaren Reaktionsbedingungen Carbonylverbindungen mit Keten in Gegenwart eines derartigen Katalysators zu den entsprechenden O-acetylierten Enolen ("Enolacetaten") reagieren.

Übliche Katalysatoren, wie etwa bekannte Amine wie etwa 1,8-Diazabicyclo[5.4.0]-undec-7-en (DBU) und 4-Dimethylamino-pyridin (DMAP) oder auch Essigsäure (pK_{S} = 4,75) sind für die gestellte Aufgabe ungeeignet.

Die Erfindung wird durch das folgende Ausführungsbeispiel näher erläutert:

### Beispiel 1

### Synthese von 1-Acetyl-2,2,6,6-tetramethyl-4-piperidon (N-Acetyl-triacetonamin, N-Acetyl-TAA)

In einem 0,5 l Vierhalskolben wurde eine Lösung von 31,0 g (0,20 Mol) TAA [Reinheit nach GC: 99,6 Fl.-%] in 250 ml trockenem Tetrahydrofuran mit 0,44 g (0,28 ml;4 mMol) Difluorphosphorsäurehydrat (HF₂PO₂x0,5 H₂O) versetzt. In diese Lösung leitete man einen Keten-Gasstrom von ca. 0,5 Mol/h ein und erwärmte die Reaktionslösung innerhalb von 1 h unter Rühren auf 46 bis 48°C (Badtemperatur: 55°C), wobei schließlich ein schwacher Ketendurchschlag festzustellen war. Nach beendeter Keten-Einleitung kühlte man auf Raumtemperatur ab, versetzte unter Rühren mit 1,0 g Natriumacetat, dekantierte von ungelöstem Salz ab und engte bei 100 bis 20 mbar und 50°C ein. Das Rohprodukt (hellbrauner Feststoff) besaß nach GC folgende Zusammensetzung (Fl.-%): 94,3 % N-Acetyl-TAA, 2,6 % TAA, 3,1 % Rest. Dies entspricht einem TAA-Umsatz von 97,4 % bei einer Selektivität von 96,8 %, bezogen auf TAA. Die Destillation bei 0,4 mbar lieferte eine N-Acetyl-TAA Hauptfraktion mit einer Reinheit von 95,3 % (29,1 g) sowie eine weitere Fraktion mit einer Reinheit von 80,4 % (2,7 g). Dies entspricht einer Ausbeute von 76 %. Die Umkristallisation der Hauptfraktion aus 60 ml n-Hexan lieferte 25,9 g gelbliches N-Acetyl-TAA mit einer Reinheit von 99,6 %.
Schmp.: 56-61 °C.

Die Untersuchung mittels ¹³C-NMR (100 Mhz, CDCl₃) lieferte folgende Ergebnisse:
- δ =: 28,41 (q, COCH₃),
30,36 (q, C(CH₃)₂),
54,07 (t, CH₂),
56,80 (s, C(CH₃)₂),
173,75 (s, NCOCH₃),
207,78 (s, C=O).

## Patentansprüche

1. Verfahren zur Herstellung von 1-Acetyl-4-piperidonen der nachstehenden Formel I 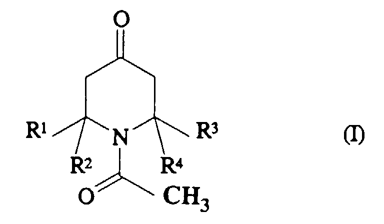 in der R¹, R², R³ und R⁴ eine C₁- bis C₆-Alkylgruppe
oder
R¹ und R² oder/und R³ und R⁴ jeweils gemeinsam eine alicyclische Kohlenwasserstoffkette mit 2 bis 5 C-Atomen bedeuten, dadurch gekennzeichnet, daß ein das entsprechende nicht-acetylierte 4-Piperidon enthaltender Bestandteil mit Keten in Gegenwart einer Säure mit einem pKₛ-Wert < 4,7 als Katalysator bei 20°C bis 120°C umgesetzt wird.

2. Verfahren nach Anspruch 1, dadurch gekennzeichnet, daß R¹, R², R³ und R⁴ C₁ bis C₃-Alkyl, insbesondere Methyl bedeuten.

3. Verfahren nach Anspruch 1 oder 2, dadurch gekennzeichnet, daß die Säure mit einem pKₛ-Wert < 4,7 in einer Menge von 0,1 bis 10 mol-% eingesetzt wird.

4. Verfahren nach einem der vorhergehenden Ansprüche, dadurch gekennzeichnet, daß die Umsetzung bei 30° bis 50°, bevorzugt im Lösungsmittel Tetrahydrofuran erfolgt.

5. Verfahren nach einem der vorhergehenden Ansprüche, dadurch gekennzeichnet, daß der Katalysator Difluorphosphorsäure ist.

## Claims

1. A process for preparing l-acetyl-4-piperidones of the formula I 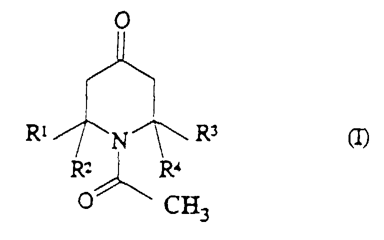 where R¹, R², R³ and R⁴ are each a C₁- to C₆-alkyl group
or
R¹ and R² together or/and R³ and R⁴ together are an alicyclic hydrocarbon chain having from 2 to 5 carbons, which comprises reacting a component comprising the corresponding nonacetylated 4-piperidone with ketene in the presence of an acid having a pKₐ < 4.7 as the catalyst at from 20°C to 120°C.

2. The process as claimed in claim 1, wherein R¹, R², R³ and R⁴ are each C₁- to C₃-alkyl, in particular methyl.

3. The process as claimed in claim 1 or 2, wherein the acid having a pKₐ < 4.7 is used in an amount of from 0.1 to 10 mol%.

4. The process as claimed in any of the preceding claims, wherein the reaction is carried out at from 30° to 50°, preferably in tetrahydrofuran as solvent.

5. The process as claimed in any of the preceding claims, wherein the catalyst used is difluorophosphoric acid.

## Revendications

1. Procédé de préparation de 1-acétyl-4-pipéridones de formule suivante I 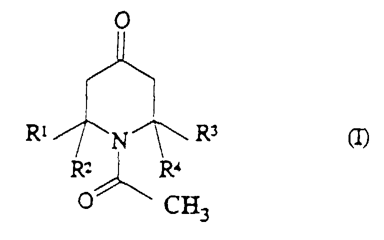 dans laquelle R¹, R², R³ et R⁴ représentent un groupement alkyle en C₁-C₆
ou
R¹ et R² ou/et R³ et R⁴ représentent à chaque fois ensemble une chaîne hydrocarbonee alicyclique ayant 2 à 5 atomes de C, caractérisé en ce qu'un composant contenant la 4-pipéridone non acylée correspondante, est mis à réagir avec du cétène, à 20-120°C en présence d'un acide ayant un pKs < 4,7 en tant que catalyseur.

2. Procéde selon la revendication 1, caractérisé en ce que R¹, R², R³ et R⁴ représentent des groupements alkyle en C₁-C₃, en particulier des groupements méthyle.

3. Procédé selon la revendication 1 ou 2 caractérisé en ce que l'acide ayant un pKs < 4,7 est utilisé en une quantité de 0,1 à 10% en moles.

4. Procédé selon l'une des revendications précédentes, caractérisé en ce que la réaction est effectuée à 30-50°C de préférence dans le solvant tétrahydrofurane.

5. Procédé selon l'une des revendications précédentes, caractérisé en ce que le catalyseur est l'acide difluorophosphorique.
